(19) Europäisches Patentamt
European Patent Office
Office européen des brevets

(11) EP 4 528 742 A1

(12) **EUROPEAN PATENT APPLICATION**
published in accordance with Art. 153(4) EPC

(43) Date of publication:
**26.03.2025 Bulletin 2025/13**

(21) Application number: **23806508.0**

(22) Date of filing: **16.01.2023**

(51) International Patent Classification (IPC):
***G16B 30/00*** *(2019.01)*

(86) International application number:
**PCT/CN2023/072456**

(87) International publication number:
**WO 2023/221546 (23.11.2023 Gazette 2023/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.05.2022 CN 202210549324**

(71) Applicant: **Shenzhen Salus Biomed Co., Ltd**
**Guangzhou, Guangdong 518107 (CN)**

(72) Inventors:
- **CHEN, Wei**
**Shenzhen, Guangdong 518107 (CN)**
- **WANG, Gufeng**
**Shenzhen, Guangdong 518107 (CN)**
- **ZHAO, Luyang**
**Shenzhen, Guangdong 518107 (CN)**

(74) Representative: **Cabinet Chaillot**
**16/20, avenue de l'Agent Sarre**
**B.P. 74**
**92703 Colombes Cedex (FR)**

# (54) BASE RECOGNITION METHOD AND APPARATUS, ELECTRONIC DEVICE, AND STORAGE MEDIUM

(57) The embodiments of the application provide a base-calling method and device, electronic equipment and a storage medium, and belongs to the technical field of recognition. The base-calling method comprises: acquiring original light intensity data of original base channels; performing crosstalk correction on the original light intensity data to obtain first corrected light intensity data; performing quantile normalization on the first corrected light intensity data to obtain initial light intensity data; performing phase correction on the initial light intensity data to obtain second corrected light intensity data; performing mean normalization on the second corrected light intensity data to obtain target light intensity data; performing intensity comparison and screening on the original base channels according to the target light intensity data to obtain a target base; and splicing multiple target bases to obtain a target base sequence. The embodiments of the application can improve the base-calling accuracy.

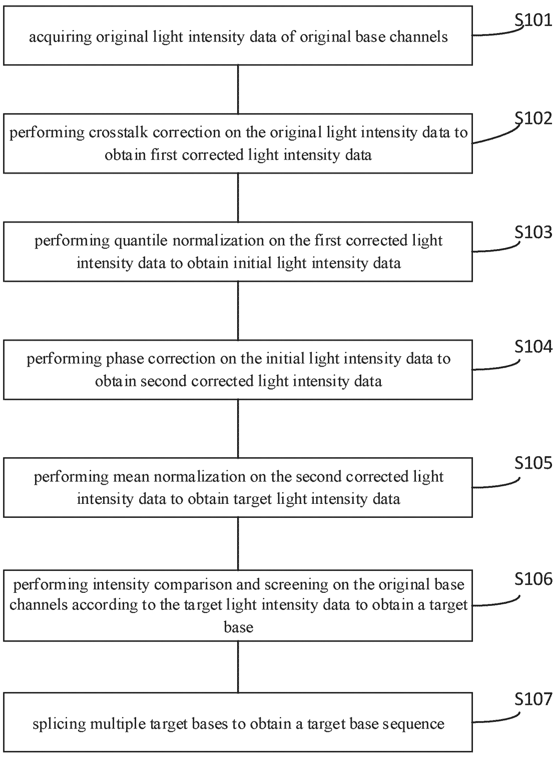


FIG. 1

EP 4 528 742 A1

## Description

### FIELD

The application relates to the technical field of recognition, and particularly relates to a base-calling method and device, electronic equipment and a storage medium.

### BACKGROUND

Existing base-calling methods often have the problem of repetitive computation at the crosstalk sampling stage and are easily disturbed by ambient noise at the intensity calculation stage, compromising the base-calling accuracy. Therefore, how to improve the base-calling accuracy is a technical issue urgently to be settled.

### SUMMARY

The main purpose of the embodiments of the application is to provide a base-calling method and device, electronic equipment and a storage medium to improve the base-calling accuracy.

In order to achieve the above purpose, a first aspect of this application embodiment provides a base-calling method, comprising:

acquiring original light intensity data of original base channels;

performing crosstalk correction on the original light intensity data to obtain first corrected light intensity data;

performing quantile normalization on the first corrected light intensity data to obtain initial light intensity data;

performing phase correction on the initial light intensity data to obtain second corrected light intensity data;

performing mean normalization on the second corrected light intensity data to obtain target light intensity data;

performing intensity comparison and screening on the original base channels according to the target light intensity data to obtain a target base; and

splicing multiple said target bases to obtain a target base sequence.

In some embodiments, the original base channels comprise a first channel and a second channel, and the performing crosstalk correction on the original light intensity data to obtain first corrected light intensity data comprises:

performing sampling on the original light intensity data by a polar coordinate sampling method to obtain first point brightness of the first channel and second point brightness of the second channel;

performing normalization on the first point brightness to obtain brightness of the first channel, and performing normalization on the second point brightness to obtain brightness of the second channel;

constructing an angle histogram according to the brightness of the first channel and the brightness of the second channel to obtain an angle sampling threshold;

classifying original bright points of the first channel and the second channel according to the angle sampling threshold to obtain target bright points; and

constructing a target crosstalk matrix according to the target bright points, and performing correction on the original light intensity data according to an inverse matrix of the target crosstalk matrix to obtain the first corrected light intensity data.

In some embodiments, the constructing an angle histogram according to the brightness of the first channel and the brightness of the second channel to obtain an angle sampling threshold comprises:

constructing the angle histogram with the brightness of the first channel as an x-axis of polar coordinates and the brightness of the second channel as a y-axis of polar coordinates;

performing feature extraction on the angle histogram to obtain an angle frequency distribution feature;

performing filtering on the angle frequency distribution feature to obtain angle peak data, wherein the angle peak data include a first angle and a second angle; and

calculating a median of the first angle and the second angle to obtain the angle sampling threshold.

In some embodiments, the performing phase correction on the initial light intensity data to obtain second corrected light intensity data comprises:

calculating a signal purity of each preset base according to a preset formula and the initial light intensity data;

performing base screening on the original base channels according to the signal purity to obtain first screening data and second screening data; and

performing phase correction on the initial light intensity data according to the first screening data and the second screening data to obtain the second corrected light intensity data.

In some embodiments, wherein the performing phase correction on the initial light intensity data according to the first screening data and the second screening data to obtain the second corrected light intensity data comprises:

performing multivariate linear regression on the first screening data and the second screening data to obtain regression parameters; and

performing correction on the initial light intensity data according to the regression parameters to obtain the second corrected light intensity data.

In some embodiments, the performing intensity brightness comparison and screening on the original base channels according to the target light intensity data to obtain a target base comprises:

extracting fluorescence intensities from the target intensity data; and

selecting the original base channel with a maximum fluorescence intensity as a target base channel, and obtaining the target base according to the target base channel.

In some embodiments, the splicing multiple said target bases to obtain a target base sequence comprises:

obtaining a preset cycle sequence; and

connecting in series the target bases in multiple cycles according to the cycle sequence to obtain the target base sequence.

In order to achieve the above purpose, a second aspect of this application embodiment provides a base-calling device, comprising:
a data acquisition module configured to acquire original light intensity data of original base channels;

a crosstalk correction module configured to perform crosstalk correction on the original light intensity data to obtain first corrected light intensity data;

a first normalization module configured to perform quantile normalization on the first corrected light intensity data to obtain initial light intensity data;

a phase correction module configured to perform phase correction on the initial light intensity data to obtain second corrected light intensity data;

a second normalization module configured to perform mean normalization on the second corrected light intensity data to obtain target light intensity data;

a base screening module configured to perform intensity comparison and screening on the original base channels according to the target light intensity data to obtain a target base; and

a base splicing module configured to splice multiple said target bases to obtain a target base sequence.

In order to achieve the above purpose, a third aspect of this application embodiment provides an electronic equipment, comprising a memory, a processor, a program stored in the memory, and a data bus for realizing a connection and communication between the processor and the memory, wherein the processor is configured to execute the program to perform the steps of the base-calling method according to the base-calling method of the first aspect are performed.

In order to achieve the above purpose, a fourth aspect of this application embodiment provides a storage medium, being a computer-readable storage medium and used for realizing computer-readable storage, wherein one or more programs are stored in the storage medium, and when the one or more programs are executed by one or more processors, the steps of the base-calling method according to the base-calling method of the first aspect are performed.

According to the base-calling method and device provided by the application, original light intensity data of original base channels are acquired, and crosstalk correction is performed on the original light intensity data to obtain first corrected light intensity data, such that a crosstalk matrix can be accurately estimated to better avoid the influence of noise, thus fulfilling good robustness. Further, quantile normalization is performed on the first corrected light intensity data to obtain initial light intensity data, phase correction is performed on the initial light intensity data to obtain second corrected light intensity data, and mean normalization is performed on the second corrected light intensity data to obtain target light intensity data, such that the light intensity data can be normalized more accurately, the base intensity of four channels can be normalized to the same dimension, and the interference of noise on intensity calculation can be reduced by phase correction, thus improving the intensity ratio calculation accuracy. Finally, intensity comparison and screening are performed on the original base channels according to the target light intensity data to obtain a target base, and multiple target bases are spliced to obtain a target base sequence, such that the target base can be determined according to target light intensity data, and target bases in multiple cycles are spliced to obtain the target base sequence, thus improving the base-calling accuracy and the precision of the generated base sequence.

## BRIEF DESCRIPTION OF THE DRAWINGS

FIG. 1 is a flow diagram of a base-calling method according to one embodiment of the application;

FIG. 2 is a flow diagram of S102 in FIG. 1;

FIG. 3 is a flow diagram of S203 in FIG. 2;

FIG. 4 is a flow diagram of S104 in FIG. 1;

FIG. 5 is a flow diagram of S402 in FIG. 3;

FIG. 6 is a flow diagram of S106 in FIG. 1;

FIG. 7 is a flow diagram of S107 in FIG. 1;

FIG. 8 is a schematic structural diagram of a base-calling device according to one embodiment of the application;

FIG. 9 is a schematic diagram of the hardware structure of electronic equipment according to one embodiment of the application.

## DESCRIPTION OF THE EMBODIMENTS

To gain a better understanding of the purposes, technical solutions and advantages of the application, the application is described in further detail below in conjunction with accompanying drawings and embodiments. It should be understood that the specific embodiments described here are merely used for explaining the invention and are not used for limiting the application.

It should be noted that although the partition of functional modules is shown in the schematic diagram of the device and logic sequences are shown in the flow diagrams, in some cases, the device may be divided into functional modules different from those shown in the schematic diagram of the device, and the steps shown or described may be performed in a sequence different from the logic sequences in the flow diagrams. The terms "first" and "second" in the description, claims and accompanying drawings are used for distinguishing similar objects and do not necessarily describe a specific sequence or precedence order.

Unless otherwise defined, all technical and scientific terms used here have the same meanings as commonly understood by those skilled in the art of the application. The terms used here are merely for the purpose of describing the embodiments of the application and are not intended to limit the application.

First, some terms involved in the application are explained:

Artificial intelligence (AI) is a new technical science studying and developing theories, methods, techniques and application systems for simulating, extending and expanding human intelligence; AI, as a branch of computer science, intends to comprehend the essence of intelligence and create a novel intelligent machine that can give response in a similar way as human intelligence; study in this field includes robots, speech recognition, image recognition, natural language processing and expert systems. AI can simulate the information process of human consciousness and thought, and may be a theory, method, technique or application system that simulates, extends and expands human intelligence, perceive environments and acquire knowledge by means of digital computers or machines controlled by digital computers and obtain an optimal result by means of the acquired knowledge.

Histogram, also referred to as quality distribution diagram, is a statistical report diagram and represents the distribution of data by a series of vertical stripes or segments with different heights. Generally, the abscissa axis indicates the type of data, the vertical axis indicates the distribution of data. The histogram is an accurate graphical representation of numerical data. It is an estimation of the probability distribution of a continuous variable (quantitative variable) and is also a bar chart. To construct a histogram, the range of values is segmented first, that is, the whole range of the values is divided into a series of intervals; then, the number of values in each interval is calculated. These values are generally specified as continuous and non-overlapped variable intervals. The intervals should be adjacent and generally (but not definitely) equal. The histogram can also be normalized to display a "relative" frequency. It can display the proportion of cases in multiple categories, and the height is equal to 1.

Linear regression is a statistical analysis method for determining a quantitative interdepend relationship between two or more variable by means of regression analysis in mathematical statistics, and is expressed as $y = w'x + e$, where e is a normal distribution, the mean value of errors of which is 0. In a case where only one independent variable and one dependent variable are included and the relationship between the independent variable and the dependent variable can be approximately represented by a straight line, the regression analysis is referred to as unitary linear regression analysis. In a case where two or more independent variables are included and dependent variables are in a linear relationship with the independent variables, the regression analysis is referred to as multivariate linear regression analysis.

Normalization is a method for simplifying computation, that is, a dimensional expression is transformed into a dimensionless expression to obtain a scalar quantity. This method is used in many computations.

Existing base-calling methods often have the problem of repetitive computation when a crosstalk matrix between channels in a cycle is calculated, and sampling of the crosstalk matrix is affected due to the non-uniform distribution of bases in some cycles, compromising the intensity correction accuracy. In addition, traditional base-calling methods are easily distributed by noisy data and outlier data when the intensity proportion is calculated, comprising the base-calling accuracy. Therefore, how to improve the base-calling accuracy and the data processing efficiency is a technical issue urgently to be settled.

The problem of repetitive computation at the crosstalk sampling stage and the disturbance of ambient noise at the intensity calculation stage compromise the base-calling accuracy. Therefore, how to improve the base-calling accuracy is a technical issue urgently to be settled.

In view of this, the embodiments of the application provide a base-calling method and device, electronic equipment and a storage medium to improve the base-calling accuracy and the data processing efficiency.

The base-calling method and device, electronic equipment and storage medium provided by the embodiments of the application are described below with reference to the following embodiments. The base-calling method provided by the embodiments of the application is described first.

In the embodiments of the application, related data may be acquired and processed based on AI. Wherein, AI is a theory, method, technique or application system that simulates, extends and expands human intelligence, perceive environments and acquire knowledge by means of digital computers or machines controlled by digital computers and obtain an optimal result by means of the acquired knowledge.

Basic AI technology generally includes, for example, sensors, dedicated AI chips, cloud computation, distributed storage, big-data processing technology, operation/interaction systems, and mechanical-electrical integration. AI software technology mainly includes computer vision technology, robot technology, biological recognition technology, speech processing technology, natural language processing technology, and machine learning/deep learning technology.

The base-calling method provided by the embodiments of the application relates to the technical field of AI, can be applied to a terminal or server, and can be software running in the terminal or server. In some embodiments, the terminal may be a smart phone, a tablet computer, a laptop computer, a desktop computer, or the like; the server may be configured as an independent physical server, or a server cluster or distributed system formed by multiple physical servers, or a cloud server which can provide basic cloud computation services such as cloud service, cloud database, cloud computation, cloud function, cloud storage, network service, cloud communication, middleware service, domain name service, security service, CDN, and big data and AI platforms; and the software may be but not limited to an application for implementing the base-calling method.

The application may be applied to various general-purpose or dedicated-purpose computer system environments or configurations, such as personal computers, server computers, handheld devices or portable devices, tablet devices, multi-processor systems, processor-based systems, set-top boxes, programmable consumer electronic equipment, network PCs, minicomputers, large computers, and distributed computation environments including any one of the systems or devices. The application can be described in the general context of a computer-executable instruction executed by a computer, such as a program module. Generally, the program module comprises a routine, program, object, module or data structure for performing a specific task or fulfilling a specific abstract data type. The application may also be practiced in distributed computation environments, in which tasks are performed by remote processing devices connected by means of a communication network. In the distributed computation environments, the program module may be located in local and remote computer storage media including storage devices.

FIG. 1 is an optional flow diagram of a base-calling method according to one embodiment of the application. As shown in FIG. 1, the base-calling method may include but not limited to S101-S107.

S101, acquiring original light intensity data of original base channels;

S102, performing crosstalk correction on the original light intensity data to obtain first corrected light intensity data;

S103, performing quantile normalization on the first corrected light intensity data to obtain initial light intensity data;

S104, performing phase correction on the initial light intensity data to obtain second corrected light intensity data;

S105, performing mean normalization on the second corrected light intensity data to obtain target light intensity data;

S106, performing intensity comparison and screening on the original base channels according to the target light intensity data to obtain a target base; and

S107, splicing multiple target bases to obtain a target base sequence.

In S101-S107 according to one embodiment of the application, original light intensity data of original base channels are acquired, and crosstalk correction is performed on the original light intensity data to obtain first corrected light intensity data, such that a crosstalk matrix can be accurately estimated to better avoid the influence of noise, thus fulfilling good robustness. Further, quantile normalization is performed on the first corrected light intensity data to obtain initial light intensity data, phase correction is performed on the initial light intensity data to obtain second corrected light intensity data, and mean normalization is performed on the second corrected light intensity data to obtain target light intensity data, such that the light intensity data can be normalized more accurately, the base intensity of four channels can be normalized to the same dimension, and the interference of noise on intensity calculation can be reduced by phase correction, thus improving the intensity ratio calculation accuracy. Finally, intensity comparison and screening are performed on the original base channels according to the target light intensity data to obtain a target base, and multiple target bases are spliced to obtain a target base sequence, such that the target base can be determined according to target light intensity data, and target bases in multiple cycles are spliced to obtain the target base sequence, thus improving the base-calling accuracy and the precision of the generated base sequence.

In some embodiments, in S101, a web crawler may be written to crawl data purposefully after a data source is set, to obtain the original light intensity data of the original base channels. The original light intensity data of the original base channels may also be acquired by other methods, which are not limited here.

It should be noted that the original base channels are light intensity channels comprising four types of bases forming DNA, and the four types of bases are adenine (A), cytosine (C), thymine (T) and guanine (G) respectively. The original light intensity data of the original base channels may be acquired from gray fluorescence images corresponding to different types of bases, four fluorescent labels are placed in one cycle to respectively label the four types of bases, and the four channels are captured with a camera to obtain gray images corresponding to different bases, that is, the four channels are captured in each cycle to obtain four gray fluorescence images. The original light intensity data include light intensities,

$I_A$, $I_T$, $I_C$ and $I_G$ , of the four channels ATCG.

Because bright points in the gray fluorescence image corresponding to each type of bases are formed by laser-excited fluorescent substances on the bases and fluorophores on the bases do not emit simplex light, the fluorophores in the four channels will be mutually influenced. Hence, it is necessary to perform crosstalk correction on the light intensity of each channel.

Referring to FIG. 2, in some embodiments, to effectively solve the problem of crosstalk between different channels, the original base channels comprise a first channel and a second channel, and S102 may comprise but not limited to S201-S205:

S201, performing sampling on the original light intensity data by a polar coordinate sampling method to obtain first point brightness of the first channel and second point brightness of the second channel;

S202, performing normalization on the first point brightness to obtain brightness of the first channel, and performing normalization on the second point brightness to obtain brightness of the second channel;

S203, constructing an angle histogram according to the brightness of the first channel and the brightness of the second channel to obtain an angle sampling threshold;

S204, classifying original bright points of the first channel and the second channel according to the angle sampling threshold to obtain target bright points; and

S205, constructing a target crosstalk matrix according to the target bright points, and performing correction on the original light intensity data according to an inverse matrix of the target crosstalk matrix to obtain the first corrected light intensity data.

To improve data processing efficiency, when inter-channel crosstalk sampling is performed, first N cycles are selected for crosstalk matrix evaluation to obtain the target crosstalk matrix; during evaluation in the subsequent cycles, the obtained target crosstalk matrix is directly used for fluorescence intensity correction. In this way, the influence of base imbalance can be reduced, and the algorithm efficiency can be improved.

In some embodiments, in S201, a degree of influence of the type of bases corresponding to one channel on the type of bases corresponding to the other channel is constructed by performing sampling on original light intensity data of any two different channels by the polar coordinate sampling method.

With the first channel (the channel corresponding to the base A, denoted as channel A) and the second channel (the channel corresponding to the base T, denoted as channel T) as an example, the proportion $R_{AT}$ of influence of the base A on the base T is calculated. Specifically, first point brightness of the first channel and second point brightness of the second channel are obtained by performing sampling on the original light intensity data by the polar coordinate sampling method, wherein the first point brightness is light brightness of all fluorescent points in the first channel, and the second point brightness is light brightness of all fluorescent points in the second channel.

In some embodiments, in S202, data preprocessing is performed on the first point brightness of the channel A and the second point brightness of the channel T, and maximum normalization and minimum normalization are performed on the first point brightness of the channel A and the second point brightness of the channel T according to a quantile 0.02 and a quantile 0.98 respectively to obtain the brightness of the first channel and the brightness of the second channel.

Referring to FIG. 3, in some embodiments, S203 may include but not limited to S301-S304:

S301, constructing the angle histogram with the brightness of the first channel as an x-axis of polar coordinates and the brightness of the second channel as a y-axis of polar coordinates;

S302, performing feature extraction on the angle histogram to obtain an angle frequency distribution feature;

S303, performing filtering on the angle frequency distribution feature to obtain angle peak data, wherein the angle peak data includes a first angle and a second angle; and

S304, calculating a median of the first angle and the second angle to obtain the angle sampling threshold.

In some embodiments, in S301, polar coordinate-based angle histogram statistics is performed on the brightness of the first channel and the brightness of the second channel, and the angle histogram is constructed with the brightness of the first channel as the x-axis of polar coordinates and the brightness of the second channel as the y-axis of polar coordinates.

In some embodiments, in S302, slope calculation is performed on each AT two-dimensional point ($x_0$, $y_0$) on the statistic angle histogram to obtain a slope corresponding to the AT two-dimensional point, and an angle Angle corresponding to the slope is obtained by calculation according to an arctan function. This process may be expressed as Angle=atan ($x_0$, $y_0$).

Further, the frequency of appearance of each angle is counted by means of a preset array Hist, wherein the array Hist may be set as needed by an actual scenario. Specifically, with 1° as a span, a corresponding frequency distribution from -45° to 135° is obtained by histogram statistics and taken as the angle frequency distribution feature corresponding to the angle histogram.

In some embodiments, in S303, to reduce the interference of noise to improve the base-calling accuracy, filtering is performed on a histogram statistic array by median filtering or other filtering methods after the angle frequency distribution feature is obtained, and peak extraction is performed on the histogram statistic array subjected to filtering. Because the AT intensity distribution shows the feature of two long arms-shaped, the histogram statistic array subjected to has two peaks, the positions of the two peaks are determined according to curve characteristics of the histogram statistic array, and angles corresponding to the positions of the two peaks are extracted and taken as angle peak data, wherein the angle peak data include a first angle D1 and a second angle S2.

In some embodiments, in S304, the median of the first angle and the second angle is calculated to obtain the angle sampling threshold D, wherein D=(D1+D2)/2.

In some embodiments, in S204, the original bright points of the first channel (channel A) and the second channel (channel T) are classified according to the angle sampling threshold D, and original bright points, an angle of which is less than the angle sampling threshold and greater than a difference between the angle sampling threshold and 180° (greater than D-180°) are taken as sampling points for calculating $R_{AT}$ (the target bright points).

In some embodiments, in S205, linear fitting is performed on all the target bright points by a least square method to obtain a linear slope, wherein the linear slope is $R_{AT}$.

Similarly, the proportion of mutual influence of every two bases is calculated by the above way, a proportion matrix M is constructed according to the series of proportions of influence of the bases, and inverse calculation is performed on the proportion matrix M to obtain a correction inverse matrix, which is the target crosstalk matrix, wherein the target crosstalk matrix $M_{inv}$ may be expressed as:

$$M_{\mathrm{inv}}=\begin{pmatrix} R_{AA} & R_{AC} & R_{AG} & R_{AT} \\ R_{CA} & R_{CC} & R_{CG} & R_{CT} \\ R_{GA} & R_{GC} & R_{GG} & R_{GT} \\ R_{TA} & R_{TC} & R_{TG} & R_{TT} \end{pmatrix}$$

The target crosstalk matrix $M_{inv}$ (4*4, four channels, four rows and four columns) is obtained by statistic analysis of different base images, and the signal intensities of the four types of bases on each fluorophore are corrected by means of the target crosstalk matrix $M_{inv}$. The original light intensity data are corrected by means of the target crosstalk matrix to obtain the first corrected light intensity data (including first corrected light intensities $I_{A1}$, $I_{T1}$, $I_{G1}$ and $I_{C1}$ of the four channels ACGA). This process may be expressed as:

$$\begin{pmatrix} R_{AA} & R_{AC} & R_{AG} & R_{AT} \\ R_{CA} & R_{CC} & R_{CG} & R_{CT} \\ R_{GA} & R_{GC} & R_{GG} & R_{GT} \\ R_{TA} & R_{TC} & R_{TG} & R_{TT} \end{pmatrix}\begin{pmatrix} I_A \\ I_C \\ I_G \\ I_T \end{pmatrix}=\begin{pmatrix} I_{A1} \\ I_{C1} \\ I_{G1} \\ I_{T1} \end{pmatrix}$$

In S201-S205 and S301-S304, the target crosstalk matrix is estimated comprehensively by means of multiple cycles, such that the influence of base imbalance can be reduced; and the target crosstalk matrix is estimated once and then directly used, such that the algorithm efficiency can be improved. Moreover, polar coordinate-based histogram statistics is used in the specific sampling process, and sampling is performed based on polar coordinates, such that the crosstalk matrix can be estimated more accurately, the influence of noise is further avoided, and the robustness is good.

In some embodiments, in S103, quantile normalization is performed on the first corrected light intensity data to obtain the initial light intensity data, such that normalization of image brightness can be realized, and an overall image normalization effect is fulfilled. The specific process may be expressed by formula (1):

$$I' = \frac{I - Per_{0.02}}{Per_{0.98} - Per_{0.02}} \quad \text{formula ( 1 )}$$

where, I is a channel intensity before normalization (the first corrected light intensity data), I' is a channel intensity after normalization (the initial light intensity data), Per is a quantile, which is 0.02 and 0.98, and $Per_{0.98}$ and $Per_{0.02}$ are respectively brightness quantiles of all fluorescent points of the channels.

It should be noted that because of fluorescence excision efficiency, fluorophores corresponding to the bases cannot be completely excised, fluorophores that fail to be excised still have certain light brightness in channel images in the next cycle, this is referred to phasing of the fluorophores. Moreover, some fluorophores have an antedating reaction, that is, some fluorophores that should react in the next cycle react in advance and show light brightness in the current cycle, this is referred to as pre-phasing of the fluorophores.

Considering the joint influence of multiple factors, to improve the phase correction accuracy, a multivariate linear regression method is used for sampling at a pre-phasing sampling and phasing sampling stage in the application.

Referring to FIG. 4, in some embodiments, S104 may comprise but not limited to S401-S403:

S401, calculating a signal purity of each preset base according to a preset formula and the initial light intensity data;

S402, performing base screening on the original base channels according to the signal purity to obtain first screening data and second screening data; and

S403, performing phase correction on the initial light intensity data according to the first screening data and the second screening data to obtain the second corrected light intensity data.

In some embodiments, in S401, the preset formula may be expressed as formula (2):

$$\text{purity} = \frac{I_{max}}{(I_A + I_T + I_C + I_G)} \quad \text{formula ( 2 )}$$

where, $I_A$, $I_T$, $I_C$ and $I_G$ are the initial light intensity data of the four channels A, T, C, G respectively; ($I_A + I_T + I_C + I_G$) is the sum of light intensities of fluorophores of the four channels in a same cycle; $I_{max}$ is a maximum channel intensity of the fluorophores at a preset sampling position, which may be obtained by comparing initial light intensities of the four channels, purity is the signal purity.

It can be understood that Purity is a quantity that is defined by the maximum channel intensity at the preset sampling position/the sum of the light intensities of the four channels; the fluorophores (the bases with the labels) have some bright points in the four channels; in a case where the brightness of the other channels is 0, Purity is 1, indicating that the called base has a high purity and will not be influenced by the other channels. In a case where the light intensities of the four channels are the same, Purity is 0.25, and it is impossible to determine the tested base corresponding to the fluorophore in the current cycle.

The signal purity of each preset base is calculated according to the preset formula, sampling by filtration is performed at the sampling position to ensure that the purity signal of the bases corresponding to the fluorophore in each cycle is not less than 0.4 and the sum of the light intensities of the fluorophore in the four channels in the same cycle is greater than 0.

In some embodiments, in S402, base screening is performed on the original base channels according to the signal purity and signal intensity; if the signal intensity of a base is maximum not in the current cycle but in the previous cycle, the base is determined as a phasing base, phasing sampling is performed on the base, and the base is added to a set of the first screening data. Similarly, if the signal intensity of a base is not maximum in the current cycle but in the next cycle of the current cycle, the base is determined as a pre-phasing base, pre-phasing sampling is performed on the base, and the base is added to a set of the second screening data.

It should be noted that to improve data processing efficiency, in the embodiments of the application, an x86 instruction set such as SSE or AVX may be used to accelerate the sampling process to increase the data processing rate under the precondition of improving the base calling accuracy, such that the base calling process can satisfy the requirement for real-time processing.

Referring to FIG. 5, in some embodiments, S403 may comprise but not limited to S501-S502:

S501, performing multivariate linear regression on the first screening data and the second screening data to obtain regression parameters; and

S502, performing correction on the initial light intensity data according to the regression parameters to obtain the second corrected light intensity data.

In some embodiments, in S501, multivariate linear regression is performed on the first screening data and the second screening data by means of a preset multivariate linear regression analysis function to obtain the regression parameters, and the specific process may be expressed as:

$$I_{current}^{channelA} \approx a + b * I_{previous}^{channelA} + c * I_{current}^{channelT} + d * I_{current}^{channelC} + e * I_{current}^{channelG} + f * I_{next}^{channelA}$$

In some embodiments, in S502, when correction is performed on the initial light intensity data according to the regression parameters, considering the cumulative effect of phasing and pre-phasing, regression fitting is performed in multiple cycles which is before or after the current cycle to improve the correction efficiency and signal accuracy. Because the accuracy will be improved with the increase in the window length of the cycle, in the embodiments of the application, a dynamic window length which increases with the increase in the cycle is used. Specifically, in the embodiments of the application, a dynamic window is used for phasing correction to satisfy the requirements of long sequencing scenarios and can improve the sequencing length and accuracy. With the increase in the cycle, phasing will increase, the cumulative effect will be enhanced, and the phasing window should be dynamically increased.

In S401-S403 and S501-S502, a variable window is used at the phasing sampling stage, such that the intensity correction is more flexible and accurate; in addition, both inter-channel correction and inter-cycle correction are performed during phasing correction, and the joint influence of multiple factors (the influence of asynchronous reaction of bases of the same channel in different cycles and the influence of base adsorption between different channels in the same cycle) are taken into account, such that the base-calling accuracy can be effectively improved.

In some embodiments, in S105, mean normalization is performed on the second corrected light intensity data to obtain the target light intensity data, such that normalization of original light intensity points can be realized, and a local image normalization effect can be fulfilled. The specific process may be expressed as by formula (3):

$$\mathrm{I}' = \frac{\mathrm{I} - \mathrm{mean}\ (\ \mathrm{I}_{\mathrm{nocall}}\ )}{\mathrm{Per}_{0.98} - \mathrm{mean}\ (\ \mathrm{I}_{\mathrm{nocall}}\ )} \quad \text{formula ( 3 )}$$

Where, I is the channel intensity before normalization (the second corrected light intensity data), I' is the channel intensity after normalization (the target light intensity data), Per is the quantile, which is 0.98, $\mathrm{Per}_{0.98}$ is the brightness quantile of all fluorescent points of the channels, the mean function mean ( $\mathrm{I}_{\mathrm{nocall}}$ ) is the calculation of a mean light intensity of all fluorescent points of non-called bases nocall in the channels, wherein the non-called bases nocall refer to bases not included in the first screening data and the second screening data.

Referring to FIG. 6, in some embodiments, S106 may comprise but not limited to S601-S602:

S601, extracting fluorescence intensities from the target intensity data; and

S602, selecting the original base channel with a maximum fluorescence intensity as a target base channel, and obtaining the target base according to the target base channel.

In some embodiments, in S601, the fluorescence intensities are extracted from the target intensity data according to a data type.

In some embodiments, in S602, the fluorescence intensities of the four original base channels (ATCG) are compared, and the type of base in reaction currently is determined according to a comparison result. Specifically, the original base channel with the maximum fluorescence intensity is selected as the target base channel, and target base is obtained according to the target base channel.

Further, a score of the target base may be calculated specifically by formula (4):

$$\mathrm{Q}_{phred} = -10 * \log_{10}(\mathrm{P}_{\varepsilon}) \quad \text{formula ( 4 )}$$

where, Q is a quality score of the target base, and $P_\varepsilon$ is an error rate of the target base.

It should be noted that if $P_\varepsilon$ is 0.1, Q is 10; if $P_\varepsilon$ is 0.01, Q is 20; if $P_\varepsilon$ is 0.001, Q is 30. That is, with the decrease of the error rate of base-calling, the quality score will increase, and the base-calling accuracy will be higher.

By performing S601-S602, the target base can be easily determined according to the fluorescence intensities, and the reaction condition of the target base is obtained by calculating the score of the target base, such that the flexibility is good.

Referring to FIG. 7, in some embodiments, S107 may comprise but not limited to S701-S702:

S701, obtaining a preset cycle sequence; and

S702, connecting in series the target bases in multiple cycles according to the cycle sequence to obtain the target base sequence.

In some embodiments, the cycle sequence obtained in S701 may be determined according to actual service requirements, and the application has not limitation in this aspect.

In some embodiments, in S702, the target bases in multiple cycles are obtained in the same way and then connected in series according to a precedence relationship in the cycle sequence to obtain the target base sequence, and the target base sequence is output in the form of a file, such that accurate base-calling is realized, and DNA sequencing is completed.

In some other embodiments, a machine learning or deep learning method may be used during calling of the target base and construction of the target base sequence to improve the base-calling accuracy and data processing efficiency.

According to the base-calling method provided by the embodiments of the application, original light intensity data of original base channels are acquired, and crosstalk correction is performed on the original light intensity data by a polar coordinate sampling method to obtain first corrected light intensity data, such that a crosstalk matrix can be accurately estimated to better avoid the influence of noise, thus fulfilling good robustness. Further, quantile normalization is performed on the first corrected light intensity data to obtain initial light intensity data, phase correction is performed on the initial light intensity data to obtain second corrected light intensity data, and mean normalization is performed on the second corrected light intensity data to obtain target light intensity data, such that the light intensity data can be normalized more accurately by two times of normalization, the base intensity of four channels can be normalized to the same dimension, and the interference of noise on intensity calculation can be reduced by phase correction, thus improving the intensity ratio calculation accuracy. Finally, intensity comparison and screening are performed on the original base channels according to the target light intensity data to obtain a target base, and multiple target bases are spliced to obtain a target base sequence, such that the target base can be determined according to target light intensity data, and target bases in multiple cycles are spliced to obtain the target base sequence, thus improving the base-calling accuracy and the precision of the generated base sequence.

Referring to FIG. 8, the embodiments of the application further provide a base-calling device for implementing the base-calling method described above, and the base-calling device comprises:

a data acquisition module 801 configured to acquire original light intensity data of original base channels;

a crosstalk correction module 802 configured to perform crosstalk correction on the original light intensity data to obtain first corrected light intensity data;

a first normalization module 803 configured to perform quantile normalization on the first corrected light intensity data to obtain initial light intensity data;

a phase correction module 804 configured to perform phase correction on the initial light intensity data to obtain second corrected light intensity data;

a second normalization module 805 configured to perform mean normalization on the second corrected light intensity data to obtain target light intensity data;

a base screening module 806 configured to perform intensity comparison and screening on the original base channels according to the target light intensity data to obtain a target base; and

a base splicing module 807 configured to splice multiple target bases to obtain a target base sequence.

The specific implementation of the base-calling device is basically the same as the specific implementation of the base-calling method and will not be repeated here.

The embodiments of the application further provide electronic equipment, comprising a memory, a processor, a program stored in the memory and capable of running on the processor, and a data bus for realizing a connection and communication between the processor and the memory, wherein when the program is executed by the processor, the base-calling method is implemented. The electronic equipment may be a tablet computer, a vehicle-mounted computer, or any other smart terminals.

Referring to FIG. 9 which illustrates a hardware structure of the electronic equipment according to another embodiment, the electronic equipment comprises:

a processor 901, which may be implemented by a central processing unit (CPU), a microprocessor, an application specific integrated circuit (ASIC), or one or more integrated circuit and is used for executing a related program to implement the technical solutions provided by the embodiments of the application;

a memory 902, which may be implemented in the form of a read only memory (ROM), a static storage device, a dynamic storage device or a random access memory (RAM), wherein the memory 902 may storage an operating system and other applications, and when the technical solutions provided by the embodiments of the application are implemented, related program codes are stored in the memory 902 and called by the processor 901 to implement the base-calling method provided by the embodiments of the application;

an input/output interface 903, which is used for realizing information input and output;

a communication interface 904, which is used for realizing communication interaction between the electronic equipment and other equipment, or realizing communication in a wired manner (for example, by means of a USB or a network cable), or realizing communication a wireless manner (for example, by means of a mobile network, WIFI, Bluetooth); and

a bus 905, which is used for transmitting information between different modules (for example, the processor 901, the memory 902, the input/output interface 903 and the communication interface 904) of the electronic equipment;

wherein, the processor 901, the memory 902, the input/output interface 903 and the communication interface 904 are in communication connection in the equipment by means of the bus 905.

The embodiments of the application further provide a storage medium, wherein the storage medium may be a computer-readable storage medium and used for realizing computer-readable storage, one or more programs are stored in the storage medium and may be executed by one or more processors to implement the base-calling method.

The memory, as a non-transient computer-readable storage medium, may be used for storing non-transient software programs and non-transient computer-executable programs. In addition, the memory may comprise a high-speed random access memory and may also comprise a non-transient memory such as at least one disk memory, a flash memory or other non-transient solid memories. In some embodiments, the memory optionally comprises a memory that is installed remotely with respect to the processor, and the remote memory may be connected to the processor by means of a network. The network, for example, includes but not limited to an internet, an intranet, a local area network, a mobile communication network and a combination thereof.

According to the base-calling method and device, electronic equipment and storage medium provided by the embodiments of the application, original light intensity data of original base channels are acquired, and crosstalk correction is performed on the original light intensity data by a polar coordinate sampling method to obtain first corrected light intensity data, such that a crosstalk matrix can be accurately estimated to better avoid the influence of noise, thus fulfilling good robustness. Further, quantile normalization is performed on the first corrected light intensity data to obtain initial light intensity data, phase correction is performed on the initial light intensity data to obtain second corrected light intensity data, and mean normalization is performed on the second corrected light intensity data to obtain target light intensity data, such that the light intensity data can be normalized more accurately by two times of normalization, the base intensity of four channels can be normalized to the same dimension, and the interference of noise on intensity calculation can be reduced by phase correction, thus improving the intensity ratio calculation accuracy. Finally, intensity comparison and screening are performed on the original base channels according to the target light intensity data to obtain a target base, and multiple target bases are spliced to obtain a target base sequence, such that the target base can be determined according to target light intensity data, and target bases in multiple cycles are spliced to obtain the target base sequence, thus improving the base-calling accuracy and the precision of the generated base sequence.

The embodiments of the application described here are used for more clearly explaining the technical solutions of the embodiments of the application and should not be construed as limitations of the technical solutions provided by the embodiments of the application. Those skilled in the art can understand that with the evolution of technology and the appearance of new application scenarios, the technical solutions provided by the embodiments of the application are also applicable to similar technical problems.

Those skilled in the art can understand that the technical solutions shown in FIGS. 1-7 should not be construed as limitations of the embodiments of the application, more or less steps may be included, some steps may be combined, or different steps may be included.

The embodiments of the device described above are merely illustrative ones, in which units described as separate components may be or may be not physically separated, that is, they may be located in the same place or distributed on multiple network units. All or part of the modules may be selected as actually needed to fulfill the purposes of the technical solutions provided by the embodiments of the application.

Those ordinarily skilled in the art can understand that the all or part of the steps of the method disclosed above and functional modules/units in the system and equipment described above may be implemented as software, firmware, hardware and appropriate combinations thereof.

Terms such as "first", "second", "third" and "fourth" (if any) in the description and accompanying drawings of the application are used for distinguishing similar objects and are not necessarily used for describing a specific sequencer or precedence order. It should be understood that these terms can be exchanged in appropriate cases to implement the embodiments of the application in other sequences different from those illustrated or described here. In addition, terms "comprise" and "provided with" and any variants thereof are intended to indicate non-exclusive inclusion. For example, a process, method, system, product or device comprising a series of steps or units is not definitely limited to the steps or units that are listed clearly, and may also include other steps or units that are not clearly listed or other inherent steps or units of the process, method, product or device.

It should be understood that in the application, "at least one" refers to one or more, and "multiple" refers to two or more. "And/or" is used for describing a relationship between related objects and indicates the presence of three relationships. For example, "A and/or B" may indicate that only A exists, only B exists and both A and B exist, wherein A and B may be in a singular form or a plural form. The character "/" generally indicates an "or" relationship between related objects. "At least one of" or similar expressions indicate any combinations of items listed, including any combinations of one or more items. For example, "at least one of a, b or c" may indicate a, b, c, a and b, a and c, b and c, or a, b and c, wherein the number of a, b and c may be one or more.

It should be understood that the device and method disclosed in the several embodiments of the application may be implemented in other ways. For example, the embodiments of the device described above are merely illustrative. For example, the division of the above units is merely a division based on logic functions, and in actual implementation, these units may be divided in other ways, for example, multiple units or modules may be combined or integrated in another system, or some features may be ignored or not implemented. In addition, coupling, direct coupling or communication connection between devices or units displayed or discussed here may refer to indirect coupling or communication connection between the devices or units by means of some interfaces, or in an electric or mechanical form.

Units that are described as separate components above may be or may not be physically separated, and components displayed as units may be or may not be physical units, that is, these units may be located in a same place or distributed in multiple network units. All or part of these units may be selected as actually needed to fulfill the purposes of the technical solutions of the embodiments of the application.

In addition, functional units in the embodiments of the application may be integrated in one processing unit or exist separately; or, two or more units may be integrated in one unit. The integrated unit may be implemented in the form of hardware or software functional unit.

The integrated unit may be stored in a computer-readable storage medium when implemented as a software functional unit and sold or used as an independent product. Based on such an understanding, the technical solutions of the application, or those that make contribution to the prior art, or all or part of the technical solutions may be embodied as a computer software product, which is stored in a storage medium, including multiple instructions used for allowing a computer (personal computer, server or network device) to perform all or part of the steps of the method provided by the embodiments of the application. The storage medium includes: a USB flash disk, a mobile hard disk drive, an ROM, an RAM, a magnetic disk, an optical disk, or other media that can store programs.

Preferred embodiments of the application are described above with reference to the accompanying drawings. but these embodiments are not intended to limit the protection scope of the embodiments of the invention. Any modifications, equivalent substitutions and improvements made by those skilled in the art without departing from the scope of the essence of the application should also fall within the protection scope of the embodiments of the application.

## Claims

**1.** A base-calling method, comprising:

acquiring original light intensity data of original base channels;
performing crosstalk correction on the original light intensity data to obtain first corrected light intensity data;
performing quantile normalization on the first corrected light intensity data to obtain initial light intensity data;
performing phase correction on the initial light intensity data to obtain second corrected light intensity data;
performing mean normalization on the second corrected light intensity data to obtain target light intensity data;
performing intensity comparison and screening on the original base channels according to the target light intensity data to obtain a target base; and
splicing multiple said target bases to obtain a target base sequence.

**2.** The base-calling method according to claim 1, wherein the original base channels comprise a first channel and a second channel, and the performing crosstalk correction on the original light intensity data to obtain first corrected light intensity data comprises:

performing sampling on the original light intensity data by a polar coordinate sampling method to obtain first point brightness of the first channel and second point brightness of the second channel;
performing normalization on the first point brightness to obtain brightness of the first channel, and performing normalization on the second point brightness to obtain brightness of the second channel;
constructing an angle histogram according to the brightness of the first channel and the brightness of the second channel to obtain an angle sampling threshold;
classifying original bright points of the first channel and the second channel according to the angle sampling threshold to obtain target bright points; and
constructing a target crosstalk matrix according to the target bright points, and performing correction on the original light intensity data according to an inverse matrix of the target crosstalk matrix to obtain the first corrected light intensity data.

**3.** The base-calling method according to claim 2, wherein the constructing an angle histogram according to the brightness of the first channel and the brightness of the second channel to obtain an angle sampling threshold comprises:

constructing the angle histogram with the brightness of the first channel as an x-axis of polar coordinates and the brightness of the second channel as a y-axis of polar coordinates;
performing feature extraction on the angle histogram to obtain an angle frequency distribution feature;
performing filtering on the angle frequency distribution feature to obtain angle peak data, wherein the angle peak data include a first angle and a second angle; and
calculating a median of the first angle and the second angle to obtain the angle sampling threshold.

**4.** The base-calling method according to claim 1, wherein the performing phase correction on the initial light intensity data to obtain second corrected light intensity data comprises:

calculating a signal purity of each preset base according to a preset formula and the initial light intensity data;
performing base screening on the original base channels according to the signal purity to obtain first screening data and second screening data; and
performing phase correction on the initial light intensity data according to the first screening data and the second screening data to obtain the second corrected light intensity data.

**5.** The base-calling method according to claim 4, **characterized in that** wherein the performing phase correction on the initial light intensity data according to the first screening data and the second screening data to obtain the second corrected light intensity data comprises:

performing multivariate linear regression on the first screening data and the second screening data to obtain regression parameters; and
performing correction on the initial light intensity data according to the regression parameters to obtain the second corrected light intensity data.

**6.** The base-calling method according to claim 1, wherein the performing intensity comparison and screening on the original base channels according to the target light intensity data to obtain a target base comprises:

extracting fluorescence intensities from the target intensity data; and

selecting the original base channel with a maximum fluorescence intensity as a target base channel, and obtaining the target base according to the target base channel.

**7.** The base-calling method according to any one of claim 1 to claim 6, wherein the splicing multiple said target bases to obtain a target base sequence comprises:

obtaining a preset cycle sequence; and
connecting in series the target bases in multiple cycles according to the cycle sequence to obtain the target base sequence.

**8.** A base-calling device, comprising:

a data acquisition module configured to acquire original light intensity data of original base channels;
a crosstalk correction module configured to perform crosstalk correction on the original light intensity data to obtain first corrected light intensity data;
a first normalization module configured to perform quantile normalization on the first corrected light intensity data to obtain initial light intensity data;
a phase correction module configured to perform phase correction on the initial light intensity data to obtain second corrected light intensity data;
a second normalization module configured to perform mean normalization on the second corrected light intensity data to obtain target light intensity data;
a base screening module configured to perform intensity comparison and screening on the original base channels according to the target light intensity data to obtain a target base; and
a base splicing module configured to splice multiple said target bases to obtain a target base sequence.

**9.** Electronic equipment, comprising a memory, a processor, a program stored in the memory, and a data bus for realizing a connection and communication between the processor and the memory, wherein the processor is configured to execute the program to perform the steps of the base-calling method according to any one of claim 1 to claim 7.

**10.** A storage medium, being a computer-readable storage medium and used for realizing computer-readable storage, wherein one or more programs are stored in the storage medium, and when the one or more programs are executed by one or more processors, the steps of the base-calling method according to any one of claim 1 to claim 7 are performed.

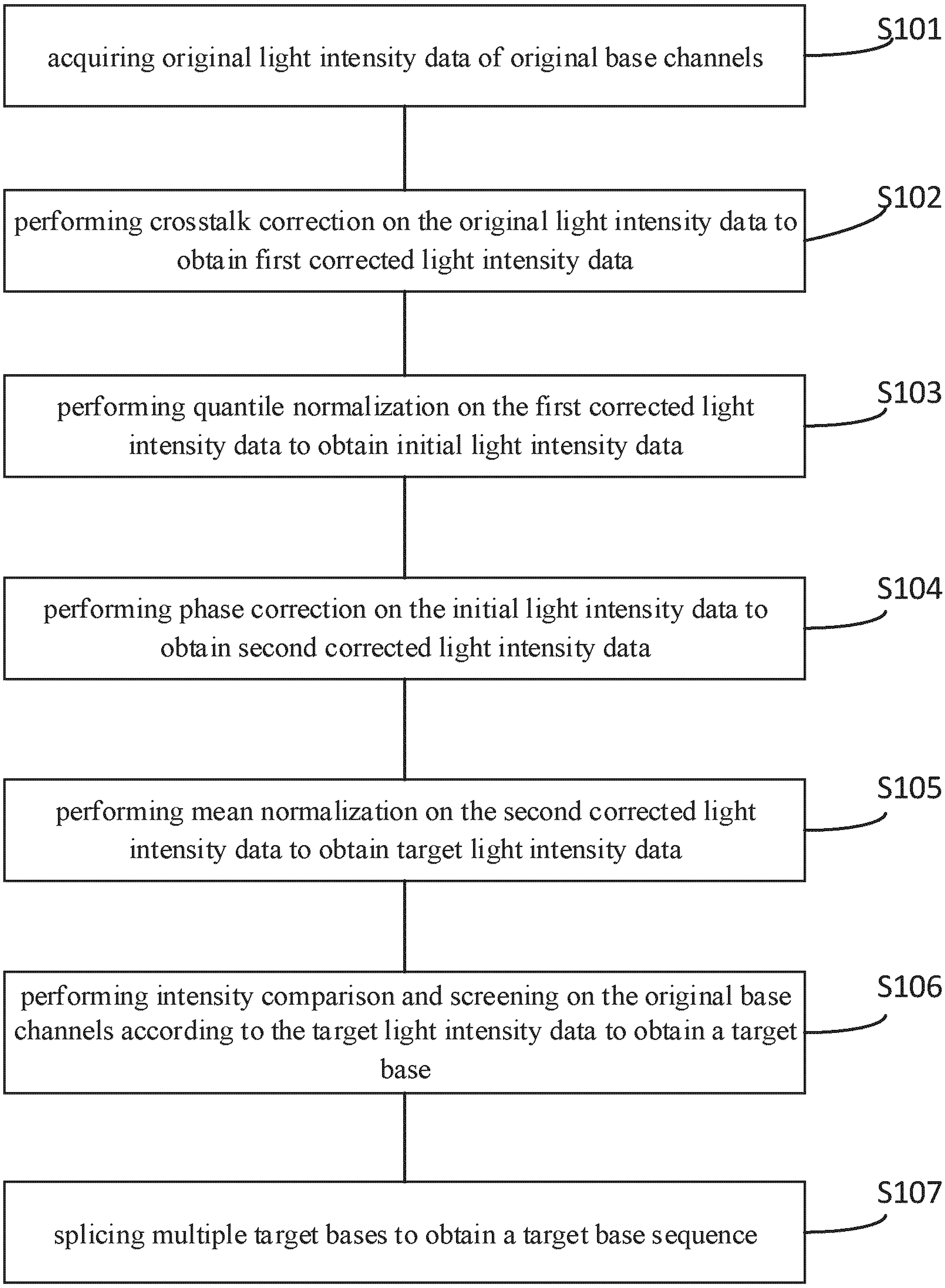
acquiring original light intensity data of original base channels
S101
performing crosstalk correction on the original light intensity data to obtain first corrected light intensity data
S102
performing quantile normalization on the first corrected light intensity data to obtain initial light intensity data
S103
performing phase correction on the initial light intensity data to obtain second corrected light intensity data
S104
performing mean normalization on the second corrected light intensity data to obtain target light intensity data
S105
performing intensity comparison and screening on the original base channels according to the target light intensity data to obtain a target base
S106
splicing multiple target bases to obtain a target base sequence
S107

FIG. 1

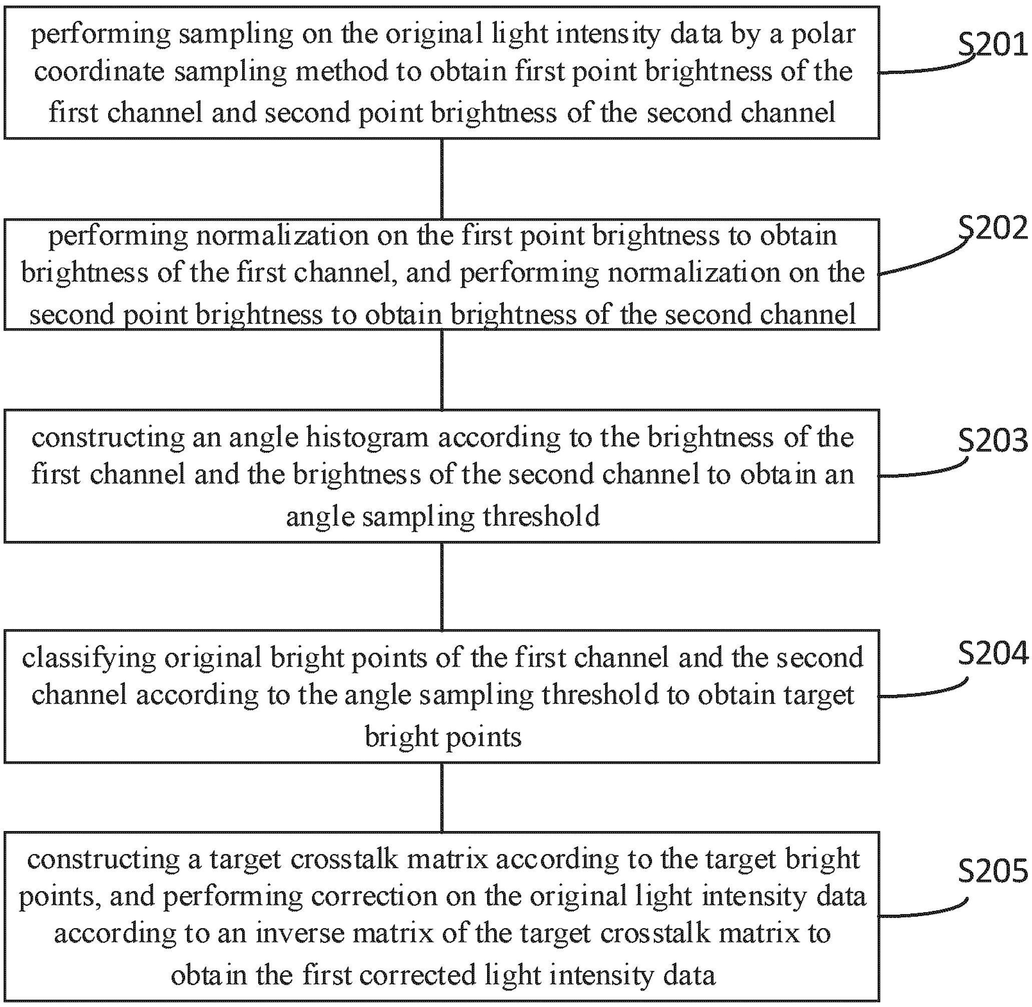
performing sampling on the original light intensity data by a polar coordinate sampling method to obtain first point brightness of the first channel and second point brightness of the second channel
S201
performing normalization on the first point brightness to obtain brightness of the first channel, and performing normalization on the second point brightness to obtain brightness of the second channel
S202
constructing an angle histogram according to the brightness of the first channel and the brightness of the second channel to obtain an angle sampling threshold
S203
classifying original bright points of the first channel and the second channel according to the angle sampling threshold to obtain target bright points
S204
constructing a target crosstalk matrix according to the target bright points, and performing correction on the original light intensity data according to an inverse matrix of the target crosstalk matrix to obtain the first corrected light intensity data
S205

FIG. 2

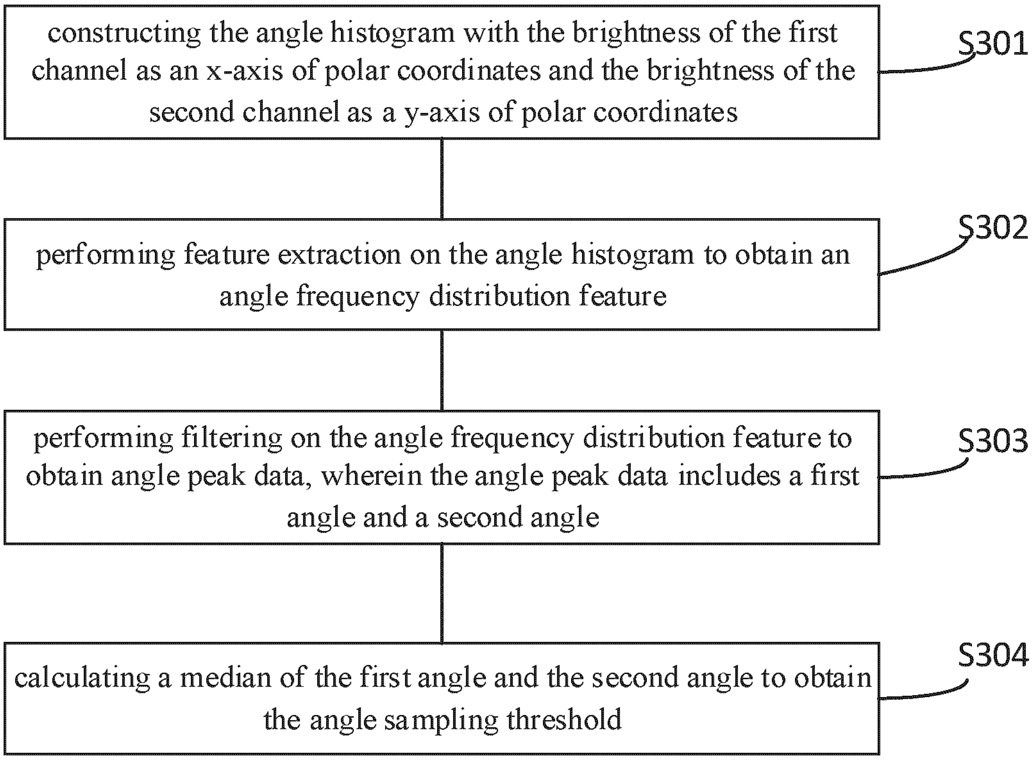
constructing the angle histogram with the brightness of the first channel as an x-axis of polar coordinates and the brightness of the second channel as a y-axis of polar coordinates
S301
performing feature extraction on the angle histogram to obtain an angle frequency distribution feature
S302
performing filtering on the angle frequency distribution feature to obtain angle peak data, wherein the angle peak data includes a first angle and a second angle
S303
calculating a median of the first angle and the second angle to obtain the angle sampling threshold
S304

FIG. 3

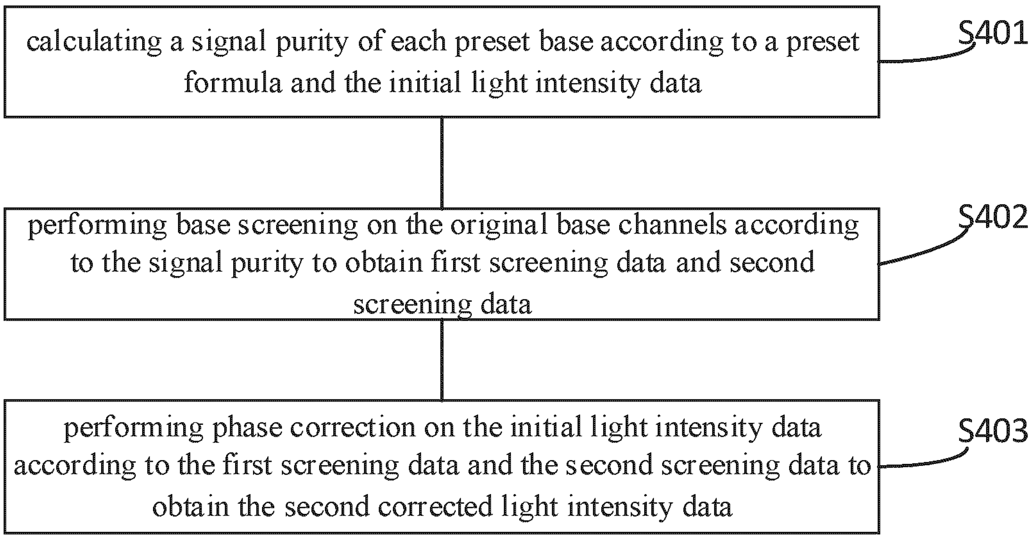
calculating a signal purity of each preset base according to a preset formula and the initial light intensity data
S401
performing base screening on the original base channels according to the signal purity to obtain first screening data and second screening data
S402
performing phase correction on the initial light intensity data according to the first screening data and the second screening data to obtain the second corrected light intensity data
S403

FIG. 4

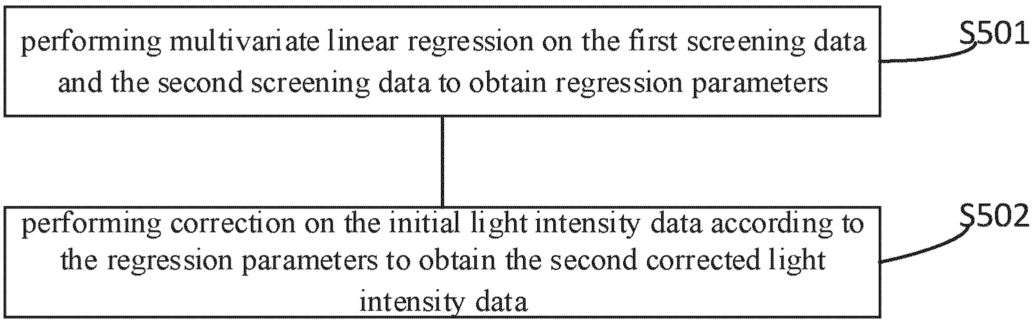


FIG. 5

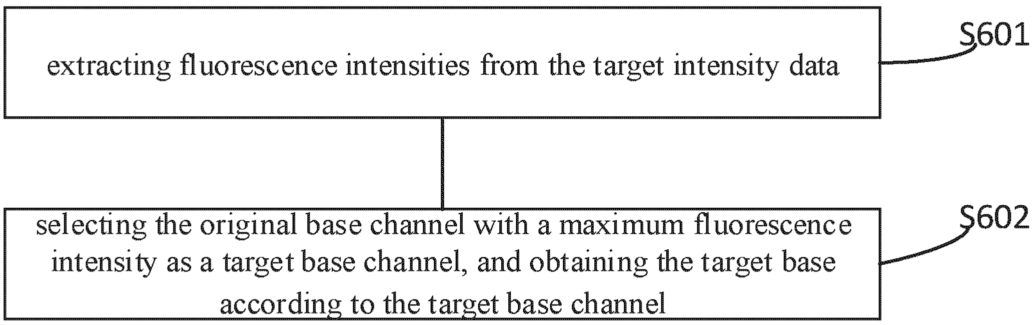


FIG. 6

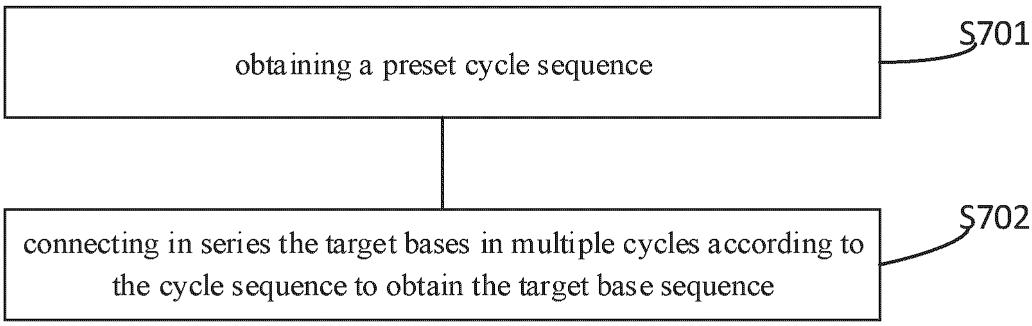


FIG. 7

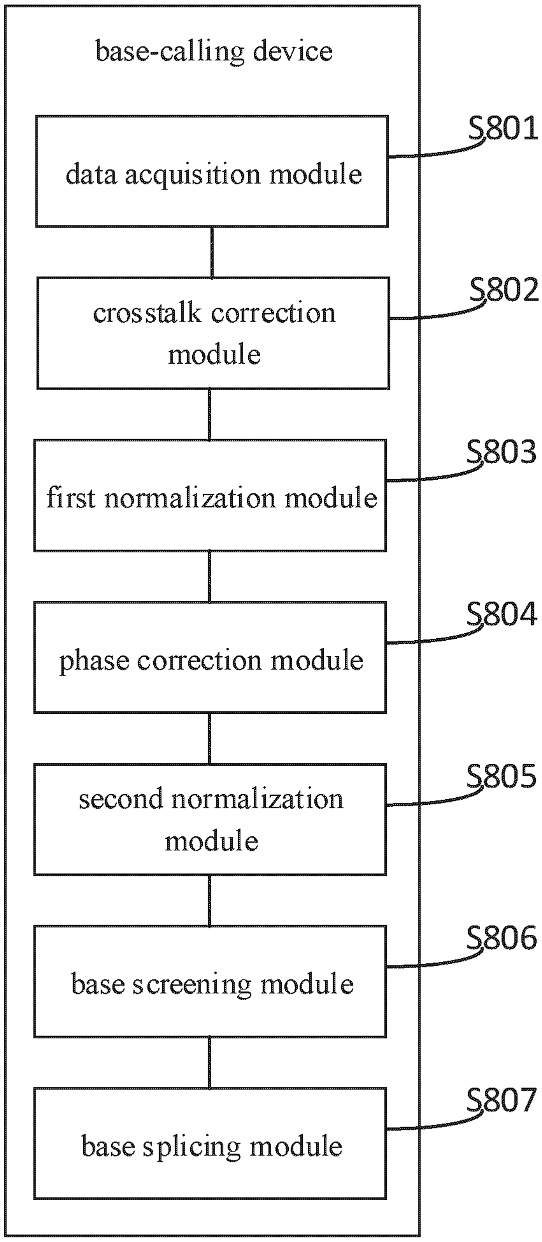
base-calling device
data acquisition module
S801
crosstalk correction module
S802
first normalization module
S803
phase correction module
S804
second normalization module
S805
base screening module
S806
base splicing module
S807

FIG. 8

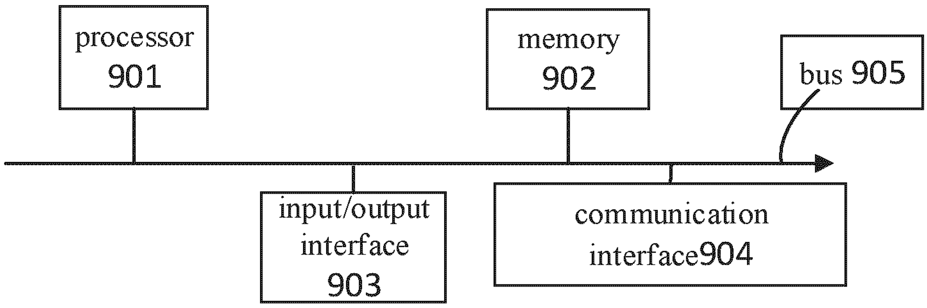
processor
901
memory
902
bus 905
input/output
interface
903
communication
interface904

FIG. 9

## INTERNATIONAL SEARCH REPORT

International application No.
**PCT/CN2023/072456**

**A. CLASSIFICATION OF SUBJECT MATTER**

G16B 30/00(2019.01)i

According to International Patent Classification (IPC) or to both national classification and IPC

**B. FIELDS SEARCHED**

Minimum documentation searched (classification system followed by classification symbols)

IPC: G16B

Documentation searched other than minimum documentation to the extent that such documents are included in the fields searched

Electronic data base consulted during the international search (name of data base and, where practicable, search terms used)

CNTXT; CNKI; ENTXT; DWPI; VEN; IEEE: 碱基, 识别, 光强, 串扰, 串色, 矫正, 相位, 通道, 亮度, 角度, 直方图; base, identification, light intensity, crosstalk, cross color, correction, phase, channel, brightness, angle, histogram

**C. DOCUMENTS CONSIDERED TO BE RELEVANT**

| Category* | Citation of document, with indication, where appropriate, of the relevant passages | Relevant to claim No. |
|---|---|---|
| PX | CN 115035952 A (SHENZHEN SAILU MEDICAL TECHNOLOGY CO., LTD.) 09 September 2022 (2022-09-09) claims 1-10 | 1-10 |
| X | CN 113012757 A (GENEMIND BIOSCIENCES CO., LTD.) 22 June 2021 (2021-06-22) description, paragraphs [0055] and [0127]-[0142] | 1-10 |
| A | CN 105989248 A (ACADEMY OF MATHEMATICS AND SYSTEMS SCIENCE, CHINESE ACADEMY OF SCIENCES) 05 October 2016 (2016-10-05) entire document | 1-10 |
| A | US 2018204085 A1 (UNIVERSITY OF PITTSBURGH-OF THE COMMONWEALTH SYSTEM OF HIGHER EDUCATION) 19 July 2018 (2018-07-19) entire document | 1-10 |

☐ Further documents are listed in the continuation of Box C.

☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 April 2023** | **19 April 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/072456**

| Patent document cited in search report | | | Publication date (day/month/year) | Patent family member(s) | | | Publication date (day/month/year) |
|---|---|---|---|---|---|---|---|
| CN | 115035952 | A | 09 September 2022 | None | | | |
| CN | 113012757 | A | 22 June 2021 | WO | 2021120715 | A1 | 24 June 2021 |
| | | | | HK | 40054464 | A0 | 25 February 2022 |
| | | | | EP | 4116402 | A1 | 11 January 2023 |
| | | | | US | 2023027811 | A1 | 26 January 2023 |
| CN | 105989248 | A | 05 October 2016 | CN | 105989248 | B | 27 November 2018 |
| US | 2018204085 | A1 | 19 July 2018 | CA | 3021538 | A1 | 15 December 2016 |
| | | | | US | 10755138 | B2 | 25 August 2020 |
| | | | | US | 2020279125 | A1 | 03 September 2020 |
| | | | | US | 11376441 | B2 | 05 July 2022 |
| | | | | EP | 3308327 | A1 | 18 April 2018 |
| | | | | EP | 3308327 | A4 | 23 January 2019 |
| | | | | CA | 3182538 | A1 | 15 December 2016 |
| | | | | HK | 1254322 | A1 | 19 July 2019 |
| | | | | JP | 2018525707 | A | 06 September 2018 |
| | | | | JP | 6910068 | B2 | 28 July 2021 |
| | | | | US | 2022323776 | A1 | 13 October 2022 |
| | | | | WO | 2016201186 | A1 | 15 December 2016 |
| | | | | CN | 107924457 | A | 17 April 2018 |
| | | | | CN | 107924457 | A8 | 18 May 2018 |
| | | | | CN | 107924457 | B | 18 March 2022 |
| | | | | HK | 1254322 | A0 | 19 July 2019 |
| | | | | JP | 021039117 | A | 11 March 2021 |

Form PCT/ISA/210 (patent family annex) (July 2022)